# FASCICULE DE BREVET EUROPEEN

(11) **EP 0 564 340 B1**
(45) Date de publication et mention de la délivrance du brevet: **22.12.1999**
(21) Numéro de dépôt: 93400798.0
(22) Date de dépôt: 29.03.1993
(51) Int. Cl.: G01N 33/53, G01N 33/573

(54) **Procédé immunochimique de détection de l'origine d'une substance produite par génie génétique et réactifs pour sa mise en oeuvre**
Verfahren zum immunochemischen Nachweis des Ursprungs einer gentechnologisch hergestellten Substanz und Reagenzien dafür
Method for the immunochemical detection of the origin of a substance produced by genetic engineering and reagents therefor

(30) Priorité: 30.03.1992 FR 9203796
(43) Date de publication de la demande: 06.10.1993
(73) Titulaire: SYNDICAT PROFESSIONNEL DES PRODUCTEURS D'AUXILIAIRES DE L'INDUSTRIE LAITIERE, SPPAIL, 75001 Paris (FR)
(72) Inventeur: Collin, Jean-Claude, F-39800 Poligny (FR); Lagarde, Gilles, F-75011 Paris (FR)
(74) Mandataire: Ores, Irène

(56) Documents cités:
- NETHERLANDS MILK AND DAIRY JOURNAL vol. 44, no. 3-4, 1990, WAGENINGEN NL pages 189 - 205 G. VAN DEN BERG ET AL. 'GOUDA CHEESEMAKING WITH PURIFIED CALF CYMOSIN AND MICROBIALLY PRODUCED CHYMOSIN.'

## Description

L'invention est relative à un procédé permettant de différencier une préparation contenant une substance obtenue par génie génétique d'une préparation de la substance naturelle correspondante, et à des réactifs pour la mise en oeuvre dudit procédé.

Les technologies de l'ADN recombinant permettent d'obtenir un grand nombre de substances qui ne pouvaient être obtenues auparavant que par extraction à partir d'animaux ou de végétaux ce qui, selon la substance considérée, peut poser certains problèmes tant au niveau du rendement d'extraction que de la pureté de la préparation obtenue.

En règle générale, les substances produites par des microorganismes transformés ont la même structure et les mêmes propriétés que les substances naturelles correspondantes et il est donc très malaisé de les en distinguer.

Or, il est parfois souhaitable d'être en mesure de procéder à cette distinction, par exemple dans un but de contrôle de qualité et/ou de détection de fraudes.

Un tel problème se pose en particulier dans l'industrie laitière, où il a été proposé d'utiliser, dans la fabrication fromagère, de la chymosine produite par génie génétique et additionnée de pepsine, à la place de présure d'origine naturelle (la présure naturelle, extraite de la caillette de jeunes bovins comprend comme constituants essentiels de la chymosine et de la pepsine).

Pour détecter des fraudes éventuelles, il est donc nécessaire de savoir distinguer de la présure naturelle les mélanges contenant la chymosine produite par génie génétique.

Or, cette distinction était jusqu'à présent considérée comme impossible, car la chymosine naturelle contenue dans la présure est absolument identique à la chymosine recombinante, par son poids moléculaire, sa séquence en acides aminés et ses propriétés immunologiques.

Un tel problème se pose, de manière plus générale, lorsqu'il s'agit de distinguer une protéine produite par génie génétique d'une protéine naturelle.

La présente Invention s'est fixé pour but la mise au point d'un procédé permettant cette distinction.

Les Inventeurs ont émis l'hypothèse que les préparations de protéines obtenues par génie génétique contenaient, même après purification, des traces de contaminants provenant du microorganisme hôte ou du milieu de culture. Ils ont recherché si ces contaminants présentaient une spécificité antigénique permettant leur identification et étaient présents en quantité suffisante pour être détectés par des méthodes immunologiques.

La présente Invention a pour objet un procédé de détection de l'origine d'une substance susceptible d'être produite dans un microorganisme hôte transformé par génie génétique, lequel procédé est caractérisé en ce qu'il comprend au moins une étape où l'on met en contact un échantillon de la substance à tester avec un anticorps dirigé contre une préparation antigénique spécifique de la culture de microorganisme hôte (c'est-à-dire, spécifique du microorganisme hôte et/ou du milieu de culture utilisé).

De façon avantageuse, le procédé selon l'Invention est caractérisé en ce que ladite préparation antigénique est susceptible d'être obtenue à partir d'un échantillon de la substance à tester, produite par génie génétique dans le microorganisme hôte, ou bien en ce que ladite préparation antigénique est susceptible d'être obtenue à partir d'une culture du microorganisme hôte délété du gène de la substance à tester.

Selon un mode de mise en oeuvre préféré de la présente Invention, la substance dont on recherche l'origine est la chymosine.

Selon un autre mode de mise en oeuvre préféré de la présente Invention, le microorganisme hôte est choisi dans le groupe constitué par *Aspergillus Niger, E. Coli* K12, et *Kluyvermyces lactis.*

L'Invention a également pour objet un réactif immunochimique pour la mise en oeuvre du procédé ci-dessus, lequel réactif est caractérisé en ce qu'il comprend au moins une préparation d'anticorps obtenue en utilisant comme immunogène une préparation antigénique spécifique du microorganisme hôte et/ou de son milieu de culture, ladite préparation antigénique spécifique du microorganisme hôte étant purifiée à partir d'un échantillon de la substance produite par génie génétique dans ledit microorganisme hôte.

La présente Invention a également pour objet un kit de dosage immunochimique pour la détermination de l'origine d'une substance susceptible d'être obtenue par génie génétique, lequel kit est caractérisé en ce qu'il comprend au moins une préparation d'anticorps telle que définie plus haut, éventuellement une préparation antigénique spécifique du microorganisme hôte et/ou de son milieu de culture, telle que définie plus haut, ainsi que des moyens de révélation usuels de la formation d'un complexe antigène-anticorps.

Toutes les méthodes de détection et de dosage immunologique peuvent convenir à la mise en oeuvre du procédé conforme à l'Invention. En particulier, des tests de type ELISA direct, sandwich ou par compétition peuvent être effectués sur des plaques de microtitration permettant ainsi des mesures quantitatives.

Pour une détermination rapide sur le terrain, on préférera des tests en bandelettes. Par exemple, avec l'utilisation d'un conjugué fabriqué avec l'antigène, une méthode ELISA compétitive peut être utilisée en fixant l'anticorps de lapin sur la bandelette et ensuite en ajoutant un mélange de conjugué et de solution inconnue à doser.

La présente Invention sera mieux comprise à l'aide du complément de description qui va suivre, qui se réfère à des exemples de préparation des réactifs conformes à l'Invention.

Il va de soi toutefois que ces exemples sont donnés uniquement à titre d'illustration de l'objet de l'Invention.

### DETERMINATION DE L'ORIGINE D'UNE PREPARATION DE CHYMOSINE

### EXEMPLE 1. - PURIFICATION DES ANTIGENES

**1° - A partir de solutions commerciales de chymosine produite par génie génétique**
   1.1. Ultrafiltration et concentration de la solution commerciale à l'aide d'une membrane de coupure à 10 000 Daltons ;
   1.2. Dialyse du produit concentré contre un tampon pipérazine 0,05M pH 5,3, afin d'éliminer la majeure partie de la chymosine par précipitation ;
   1.3. Chromatographie sur colonne d'hydrophobicité de la fraction non précipitée (Colonne de Phényl-superose de chez PHARMACIA). L'élution est effectuée par un tampon phosphate de potassium 0,1 M, pH 7 et un gradient NaSO₄ 1,7 M à 0 M. On élimine les fractions qui coagulent le lait, on récupère les autres, qui sont réunies.
   1.4. Lyophilisation des fractions obtenues précédemment ;
   1.5 Le lyophilisat est chromatographié sur un gel de perméation (Colonne superose 12 de chez PHARMACIA) ; l'élution est effectuée par tampon pipérazine HCl 0,05 M, pH 5,3. On élimine les fractions qui coagulent le lait, on recueille les autres.
   1.6. Les fractions recueillies sont dialysées contre une solution 0,01 M de carbonate acide d'ammonium, puis lyophilisées.
**2° - A partir de cultures de souches microbiennes hôtes délétées**
   Les antigènes sont préparés à partir des protéines excrétées dans le milieu de culture, concentrées par ultrafiltration. Le concentrat est ensuite lyophylisé.

### EXEMPLE 2 - PROTOCOLE D'OBTENTION D'ANTICORPS POLYCLONAUX CHEZ LE LAPIN

. Des lapines de race vulgaire de 2 à 3 mois sont choisies (environ 3 kg).
. La première injection se fait par voie intradermique avec une des préparations antigéniques obtenue comme décrit à l'Exemple 1 (200 µg de protéines) dissous dans 1 ml de solution contenant, à volumes égaux, de l'eau physiologique et de l'adjuvant complet de Freund.
. Les injections suivantes se font toutes les trois semaines avec la même quantité de préparation antigénique en émulsion dans l'adjuvant incomplet de Freund.
. Les prélèvements de sang sont réalisés dix jours après l'injection
. La qualité de l'anticorps est déterminée par une méthode ELISA directe en fixant l'antigène sur la plaque, puis le sérum de lapin et un conjugué commercial anti-Ig de lapin.

### EXEMPLE 3 - PROTOCOLE D'OBTENTION D'ANTICORPS POLYCLONAUX CHEZ LA POULE

. La première injection intramusculaire est réalisée avec 200 µg de protéines en solution dans 1 ml d'un mélange contenant, à volumes égaux, de l'eau physiologique et de l'adjuvant complet de Freund.
. Les injections suivantes se font toutes les trois semaines avec 200 µg de protéines en émulsion dans l'adjuvant incomplet de Freund.
. Les oeufs sont retenus dix jours après l'injection. Les anticorps sont extraits du jaune d'oeuf par précipitation avec 1,2% (P/V) de polyéthylène glycol.

### EXEMPLE 4 - DETERMINATION DE L'ORIGINE D'UNE PREPARATION D'ENZYMES DE COAGULATION DU LAIT

### Méthode ELISA directe sur bandelette

a) Dépôt d'une goutte de 2 à 4 µl d'un échantillon non dilué à tester dans un cercle dessiné sur la bandelette et noté A ;
b) Dépôt d'une goutte de témoin positif (solution commerciale de chymosine obtenue par génie génétique ou préparation d'antigène obtenue comme décrit à l'exemple 1) dans un cercle dessiné sur la bandelette et noté B ;
c) Attendre 10 min ;
d) Tremper pendant 10 min la bandelette dans la solution de saturation (poudre de lait à 0,5%, gélatine 0,5%, Tween® 20 à 0,1%) ;
e) Laver la bandelette avec un tampon phosphate pH 7,2 contenant du Tween® 20 à 0,05% (solution de lavage) ;
f) Tremper la bandelette 10 min dans une solution diluée au 1/200 eme d'anticorps de lapin préparée comme décrit à l'exemple 2 ;
g) Laver la bandelette avec la solution de lavage ;
h) Tremper 10 min la bandelette dans une solution au 1/200 eme le conjugué anti-immunoglobulines de lapin marqué à la peroxydase;
i) Rincer soigneusement la bandelette avec la solution de lavage ;
j) Tremper la bandelette pendant 5 à 10 min dans la solution de substrat [diaminobenzidine (DAB)] ;
k) Lire le résultat en comparant la coloration brune du dépôt A avec celle du dépôt B.

### Méthode ELISA sandwich sur bandelette

a) Utilisation d'une bandelette sensibilisée dans deux cercles notés A et B avec des immunoglobulines de poule ;
b) Dépôt d'une goutte de solution inconnue (échantillon à analyser) dans le cercle A ;
c) Dépôt d'une goutte de témoin positif dans le cercle B ;
d) Attendre 10 min ;
e) Laver la bandelette avec la solution de lavage ;
f) Identique à la méthode précédente pour les points f à k.

### Méthode ELISA directe sur plaque

a) On ajoute successivement dans chaque puits d'une plaque de microtitration, 100 µl d'une solution à tester éventuellement diluée tampon carbonate 0,1 M pH 9.6.
b) 100 µl d'une solution témoin positif diluée dans le même tampon ;
   Incubation : 2h à 37 °C ;
   3 lavages tampon phosphate 0,15 M Nacl, Tween® 20 à 0,05% (PBS Tween).
c) Saturation des puits avec 100 µl sérum albumine bovine 1% diluée dans tampon PBS Tween ;
   Incubation : 1 h à 37°C.
d) Après 3 lavages dans PBS Tween, on ajoute 100 µl sérum lapin dilué dans PBS Tween ;
   Incubation : 1h30 à 37°C.
e) Après 3 lavages dans PBS Tween, on ajoute 100 µl conjugué anti IgG lapin, couplé à phosphatase alcaline ;
   Incubation : 1h30 à 37 °C.
f) Après 3 lavages dans PBS Tween, on ajoute 100 µl paranitrophényl phosphate à 1 mg/ml ;

On procède à la lecture à 405 mn après 1h environ, en fonction de l'intensité de la coloration.

### RESULTATS

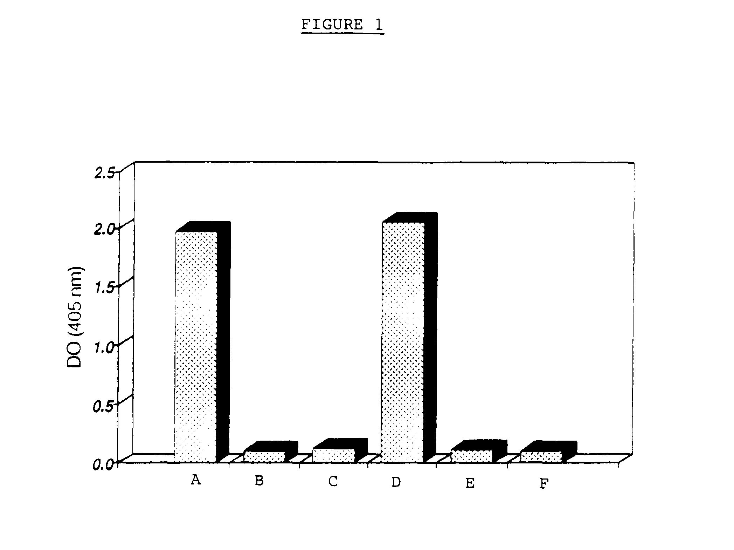

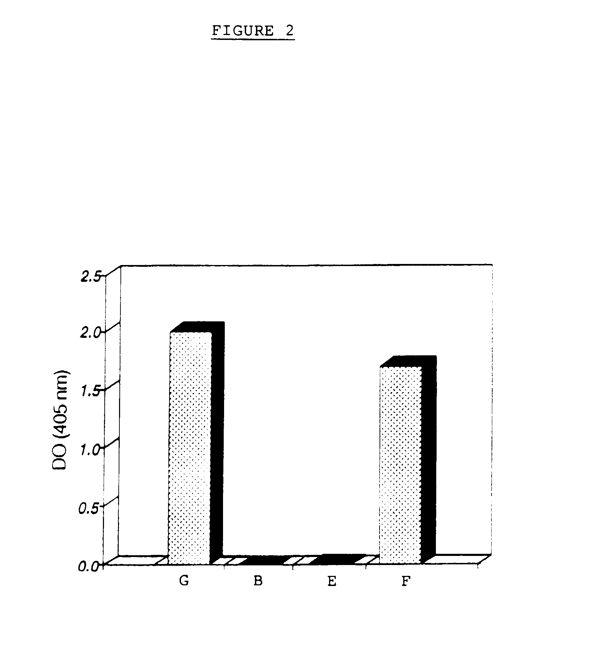

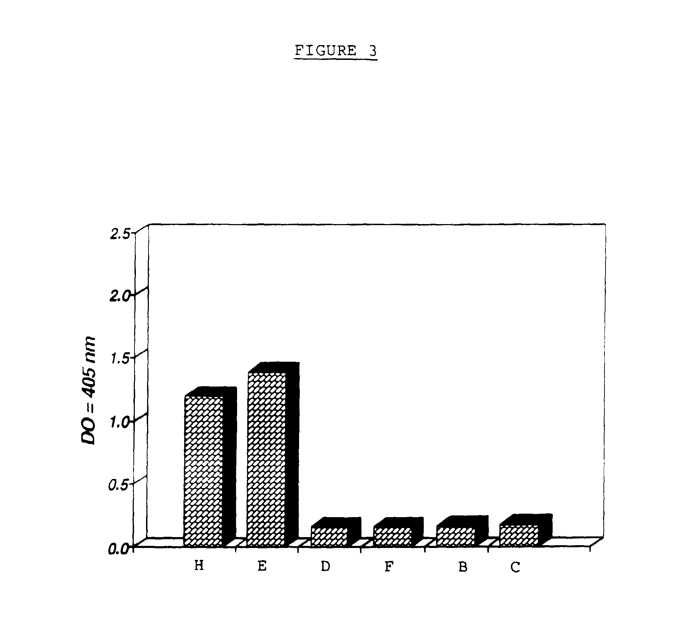

Les figures 1 à 3 illustrent les résultats obtenus par la méthode ELISA directe sur plaque, en utilisant comme antigène à tester, différentes préparations d'enzymes de coagulation, et en utilisant comme antigènes témoin, soit une préparation purifiée à partir d'une solution commerciale de chymosine recombinante, soit une préparation obtenue à partir d'une culture de microorganismes délétés du gène de la chymosine.

### a) Antigènes testés

A) Préparation d'antigène obtenu par élimination de la chymosine d'une préparation de CHYMOGEN®.
B) Préparation de présure naturelle
C) Préparation de chymosine obtenue à partir de chymosine animale, purifiée par échange d'ions (Laboratoires GRANDAY-ROGER, SANOFI BIO-INDUSTRIE)
D) Préparation de chymosine CHYMOGEN®/CHR HANSENS vendue par la Société BOLL, produite par une souche de *Aspergillus Niger*.
E) Préparation de chymosine CHYMAX® (vendue par PFIZER) produite par une souche de *E. Coli* K12
F) Préparation de chymosine MAXIREN® (vendue par GIST-BROCADES) produite par une souche de *Kluveromyces lactis*.

### b) Antigènes d'immunisation et anticorps

La figure 1 représente les résultats obtenus avec l'antisérum CGB23 ter obtenu en utilisant comme immunogène l'antigène A ci-dessus, lequel antisérum est dilué au 1/100.

La figure 2 représente les résultats obtenus avec l'antisérum CGG13 obtenu en utilisant comme immunogène un antigène produit par une souche de *Kluveromyces lactis* délétée du gène codant pour la chymosine (antigène G), lequel antisérum est dilué au 1/200

La figure 3 représente les résultats obtenus avec l'antisérum CGP52 obtenu en utilisant comme immunogène un antigène produit par une souche *d'E. Coli* K12, délétée du gène codant pour la chymosine (antigène H), lequel antisérum est dilué au 1/100.

Pour chacune de ces figures, en abscisse sont représentées les solutions antigéniques à tester diluées au 1/10. Les solutions témoin (antigènes d'immunisation) sont à une concentration de 500 ng/ml. En ordonnée est indiquée la DO₄₀₅.

## Revendications

1. Procédé de détection de l'origine d'une substance susceptible d'être produite dans un microorganisme hôte transformé par génie génétique, lequel procédé est caractérisé en ce qu'il comprend au moins une étape où l'on met en contact un échantillon de la substance à tester avec un anticorps dirigé contre une préparation antigénique spécifique du microorganisme hôte et/ou de son milieu de culture.

2. Procédé selon la revendication 1, caractérisé en ce que ladite préparation antigénique est susceptible d'être obtenue à partir d'un échantillon de la substance à tester, produite par génie génétique dans le microorganisme hôte.

3. Procédé selon la revendication 1, caractérisé en ce que ladite préparation antigénique est susceptible d'être obtenue à partir d'une culture du microrganisme hôte délété du gène de la substance à tester.

4. Procédé selon une quelconque des revendications 1 à 3, caractérisé en ce que la substance à tester est la chymosine.

5. Procédé selon l'une quelconque des revendications 1 à 4, caractérisé en ce que le microorganisme hôte est choisi dans le groupe constitué par *Aspergillus niger, E. Coli* K12, et *Kluyvermyces* lactis.

6. Réactif immunochimique pour la mise en oeuvre du procédé selon l'une quelconque des revendications 1 à 5, lequel réactif est caractérisé en ce qu'il comprend au moins une préparation d'anticorps obtenue en utilisant comme immunogène une préparation antigénique spécifique du microorganisme hôte et/ou de son milieu de culture, ladite préparation antigénique spécifique du microorganisme hôte étant purifiée à partir d'un échantillon de la substance produite par génie génétique dans ledit microorganisme hôte.

7. Kit de dosage immunochimique pour la détermination de l'origine d'une substance susceptible d'être obtenue par génie génétique, caractérisé en ce qu'il comprend au moins un réactif selon la revendication 6, éventuellement une préparation antigénique spécifique du microorganisme hôte et/ou de son milieu de culture, ainsi que des moyens de révélation usuels de la formation d'un complexe antigène-anticorps.

## Patentansprüche

1. Verfahren zum Nachweis des Ursprungs einer Substanz, die in einem gentechnisch transformierten Wirtsmikroorganismus produziert werden kann, dadurch **gekennzeichnet**, daß es mindestens eine Stufe umfaßt, im Verlauf derer man eine Probe der zu testenden Substanz mit einem gegen ein für den Wirtsmikroorganismus und/oder sein Kulturmedium spezifisches antigenes Präparat gerichteten Antikörper in Kontakt bringt.

2. Verfahren nach Anspruch 1, dadurch **gekennzeichnet**, daß das antigene Präparat von einer Probe der zu testenden Substanz erhalten werden kann, die gentechnisch in dem Wirtsmikroorganismus produziert wurde.

3. Verfahren nach Anspruch 1, dadurch **gekennzeichnet**, daß das antigene Präparat von einer Kultur des Wirtsmikroorganismus, bei dem das Gen der zu testenden Substanz deletiert wurde, erhalten werden kann.

4. Verfahren nach einem der Ansprüche 1 bis 3, dadurch **gekennzeichnet**, daß die zu testende Substanz Chymosin ist.

5. Verfahren nach einem der Ansprüche 1 bis 4, dadurch **gekennzeichnet**, daß der Wirtsmikroorganismus aus der Gruppe bestehend aus *Aspergillus niger, E. Coli K12* und *Kluyvermyces lactis* ausgewählt wird.

6. Immunchemisches Reagens zur Durchführung des Verfahrens nach einem der Ansprüche 1 bis 5, dadurch **gekennzeichnet**, daß es mindestens ein Antikörperpräparat, erhalten unter Verwendung eines für den Wirtsmikroorganismus und/oder sein Kulturmedium spezifischen antigenen Präparats als Immunogen, enthält, wobei das für den Wirtsmikroorganismus antigene Präparat aus einer Probe der gentechnisch in dem Wirtsmikroorganismus produzierten Substanz gereinigt wurde.

7. Kit zur immunchemischen Bestimmung zur Bestimmung des Ursprungs einer Substanz, die gentechnisch erhalten werden kann, dadurch **gekennzeichnet**, daß es mindestens ein Reagens nach Anspruch 6, gegebenenfalls ein antigenes Präparat, das für den Wirtsmikroorganismus und/oder sein Kulturmedium spezifisch ist, sowie übliche Mittel zur Detektion der Bildung eines Antigen-Antikörper-Komplexes enthält.

## Claims

1. A process for detecting the origin of a substance capable of being produced in a host microorganism transformed by genetic engineering, which process is characterised in that it comprises at least one step in which a sample of the substance to be tested is brought into contact with an antibody directed against a specific antigen preparation of the host microorganism and/or its culture medium.

2. A process according to claim 1, characterised in that the said antigen preparation is capable of being obtained from a sample of the substance to be tested, produced by genetic engineering in the host microorganism.

3. A process according to claim 1, characterised in that the said antigen preparation is capable of being obtained from a culture of the host microorganism from which has been deleted the gene of the substance to be tested.

4. A process according to any one of claims 1 to 3, characterised in that the substance to be tested is rennin.

5. A process according to any one of claims 1 to 4, characterised in that the host microorganism is chosen from the group composed of *Aspergillus niger*, *E. Coli K12* and *Kluyvermyces lactis*.

6. An immunochemical reagent for carrying out the process according to any one of claims 1 to 5, which reagent is characterised in that it comprises at least one antibody preparation obtained using, as immunogen, a specific antigen preparation of the host microorganism and/or its culture medium, the said specific antigen preparation of the host microorganism being purified from a sample of the substance produced by genetic engineering in the said host microorganism.

7. An immunochemical assay kit for determining the origin of a substance capable of being obtained by genetic engineering, characterised in that it comprises at least one reagent according to claim 6, optionally a specific antigen preparation of the host microorganism and/or its culture medium, and usual means of detecting the formation of an antigen-antibody complex.
